# EUROPEAN PATENT APPLICATION

(11) **EP 4 745 988 A1**
(43) Date of publication of application: **20.05.2026**
(21) Application number: 24839417.3
(22) Date of filing: 14.06.2024
(51) Int. Cl.: G16H 50/20

(54) **PATIENT CARE PLAN DETERMINATION SYSTEM AND PATIENT CARE PLAN DETERMINATION METHOD**

(30) Priority: 10.07.2023 JP 2023113131
(71) Applicant: HITACHI HIGH-TECH CORPORATION, Tokyo 105-6409 (JP)
(72) Inventor: LI Zisheng, Tokyo 100-8280 (JP); HAYASHI Kaede, Tokyo 105-6409 (JP); AKIKAWA Ryota, Tokyo 105-6409 (JP); SUZUKI Mayumi, Tokyo 105-6409 (JP); OGINO Masahiro, Tokyo 100-8280 (JP)
(74) Representative: Strehl & Partner mbB
(86) International application number: PCT/JP2024/021783
(87) International publication number: WO 2025/013521

(57) **Abstract**

A highly valid test is recommended based on a result at each timing of testing, diagnosis, treatment, and follow-up monitoring within a holistic patient management workflow. A patient care plan determination system of the disclosure includes: a medical information server configured to store at least attribute information of a plurality of patients and a test result; an information terminal configured to provide a patient management policy indicating a classification of a target patient; and a computer configured to acquire predetermined information from the medical information server and the information terminal and determine a candidate test item related to a disease of the target patient. The computer executes processing of estimating a test value of a future candidate test item of the target patient by applying the patient attribute information of the target patient, a test result of the target patient, and the patient management policy of the target patient to a test plan trained model for calculating the test value for each test item according to the patient management policy, the test value indicating information capable of specifying a disease, and processing of outputting the candidate test item of the target patient and the test value (see FIG. 6).

## Description

### Technical Field

The present disclosure relates to a patient care plan determination system and a patient care plan determination method.

### Background Art

A task of a doctor in test ordering is to integrate and interpret a large amount of clinical information and select an appropriate test for each patient. Specifically, a doctor (i) collects patient information by examining a patient, lists suspected diseases based on an examination result, and prioritizes the diseases, (ii) selects and executes a necessary test from all possible tests in consideration of a degree of contribution to disease identification and a patient load (issues a test order), (iii) interprets a result value of the test and updates a suspected disease list, and (iv) repeats steps (ii) and (iii) until a disease is identified.

In order to accurately obtain such a test order, it is often necessary for a doctor to have a large amount of experience. Therefore, for example, PTL 1 proposes a system that supports planning of a test and/or a treatment to be implemented on a predetermined patient by displaying case information registered in a database on a screen.

### Citation List

### Patent Literature

PTL 1: JP2007-287027A

### Summary of Invention

### Technical Problem

It is extremely difficult to provide a patient with an appropriate test based on positioning of testing within the holistic patient management continuum, such as an initial visit, a revisit, follow-up during hospitalization, and emergency, and desired clinical utility. A treatment and a diagnosis for a patient in a hospital are performed according to a management policy. Herein, the management policy refers to information defining a test, a diagnose, and a treatment to be implemented based on patient classification (patient positioning), such as an initial visit patient, a revisit patient, a hospitalized patient, a target patient under follow-up monitoring, and an emergency patient.

The system disclosed in PTL 1 can merely refer to past cases registered in a database, and cannot support a test and a treatment according to a patient management policy. In other words, even when symptoms are the same, a testing method and a treatment method are different depending on patient positioning such as an initial visit or a revisit, so that an appropriate test and treatment cannot be implemented by simply referring to past cases. Therefore, when there is a system that recommends a highly valid test item at an appropriate timing in consideration of the holistic patient management continuum, an appropriate test can be implemented on various patients regardless of an experience level of the doctor.

In view of such circumstances, the disclosure proposes a technique that recommends a highly valid test based on a result at each timing of testing, diagnosis, treatment, and follow-up monitoring within a holistic patient management workflow.

### Solution to Problem

In order to solve the above problem, as an example, the disclosure proposes a patient care plan determination system for determining at least a candidate test item related to a disease of a target patient. The system includes: a medical information server configured to store at least attribute information and a test result of a plurality of patients; an information terminal configured to provide a patient management policy indicating a classification of the target patient; and a computer configured to acquire predetermined information from the medical information server and the information terminal and determine the candidate test item related to the disease of the target patient. The computer executes processing of acquiring, from the medical information server, attribute information of the target patient and a patient management policy of the target patient, processing of estimating a test value of a future candidate test item of the target patient by applying the patient attribute information of the target patient, a test result of the target patient, and the patient management policy of the target patient to a test plan trained model for calculating the test value for each test item according to the patient management policy, the test value indicating information capable of specifying a disease, and processing of outputting the candidate test item of the target patient and the test value.

Additional features related to the present disclosure will be clarified based on the description of the present description and the accompanying drawings. Aspects of the present disclosure may be achieved and implemented using elements, combinations of various elements, the following detailed description, and accompanying claims.

The description of the present description is merely a typical illustration and does not limit the scope of the claims or application examples of the present disclosure in any sense.

### Advantageous Effects of Invention

According to the technique of the disclosure, a highly valid test can be recommended based on a result at each timing of testing, diagnosis, treatment, and follow-up monitoring within a holistic patient management workflow.

### Brief Description of Drawings

[FIG. 1] FIG. 1 is a diagram illustrating a schematic configuration example of an information processing system (also referred to as a patient care plan determination system) 10 according to the present embodiment.
[FIG. 2] FIG. 2 is a diagram illustrating a functional configuration example (software configuration example) of the information processing system (patient care plan determination system) 10 according to a first embodiment.
[FIG. 3] FIG. 3 is a diagram illustrating a data flow in training processing executed by a test training unit 300 of a test planning unit 1012.
[FIG. 4] FIG. 4 is a flowchart illustrating details of training parameter determination processing according to the first embodiment.
[FIG. 5] FIG. 5 is a flowchart illustrating details of processing of determining (training) a threshold of a test value when constructing a trained model.
[FIG. 6] FIG. 6 is a diagram illustrating a data flow in prediction processing executed by a test prediction unit 600 of the test planning unit 1012.
[FIG. 7] FIG. 7 is a flowchart illustrating details of determination of a test item t+1 at time point t+1 and estimation of a probability of being diagnosed with a disease t+1 (diagnosis probability of the disease t+1) according to the first embodiment.
[FIG. 8] FIG. 8 is a diagram illustrating a configuration example of patient information 800 according to the present embodiment (common to the first embodiment and a second embodiment).
[FIG. 9] FIG. 9 is a flowchart illustrating test cost estimation processing executed by the test planning unit 1012.
[FIG. 10] FIG. 10 is a diagram illustrating a configuration example of a test order GUI 1000 displayed on a display screen of an information terminal 110 according to the first embodiment.
[FIG. 11] FIG. 11 is a diagram illustrating a functional configuration example (software configuration example) of the information processing system (patient care plan determination system) 10 according to the second embodiment.
[FIG. 12] FIG. 12 is a diagram illustrating a detailed internal logical configuration example of a medical service planning unit 1100 and the test planning unit 1012 in the patient care plan determination system 10 of the second embodiment.
[FIG. 13] FIG. 13 is a diagram illustrating a data flow in training processing executed by a medical service training unit t1301_1 to a medical service training unit t+N1301_N of the medical service planning unit 1100.
[FIG. 14] FIG. 14 is a flowchart illustrating training parameter determination processing (trained model generation processing) executed by the medical service training unit t1301_1 to the medical service training unit t+N_1301_N in the second embodiment.
[FIG. 15] FIG. 15 is a diagram illustrating a data flow in medical service prediction processing executed by a medical service prediction unit 1500 of the medical service planning unit 1100.
[FIG. 16] FIG. 16 is a flowchart illustrating processing of estimating a plurality of patterns of a patient management policy, a medical service method, and patient disease information from time point t+1 (time step t+1) to time point t+N (time step t+N) according to the second embodiment.
[FIG. 17] FIG. 17 is a diagram illustrating a data flow in training processing executed by a test training unit 1700 of the test planning unit 1012 of the second embodiment.
[FIG. 18] FIG. 18 is a flowchart illustrating details of training parameter determination processing according to the second embodiment.
[FIG. 19] FIG. 19 is a diagram illustrating a configuration example of an output screen (user interface: UI) 1900 of a medical service plan and a test plan according to the second embodiment.
[FIG. 20] FIG. 20 is a diagram illustrating a configuration example of a test order GUI 2000 displayed on a display screen of the information terminal 110 according to the second embodiment.

### Description of Embodiments

The present embodiment discloses an information processing system (patient care plan determination system) that predicts a medical service plan including each process and result of a patient management phase, testing, diagnosis, treatment, and follow-up monitoring based on patient information, and estimates a test item necessary for executing the medical service plan.

Hereinafter, an embodiment of the present disclosure will be described with reference to the accompanying drawings. In the accompanying drawings, functionally the same elements may be denoted by the same reference numerals. The accompanying drawings show specific embodiments and implementation examples according to the principle of the disclosure, but the embodiments and the implementation examples are provided for understanding the present disclosure and are not by no means to be used to limit the present disclosure.

Embodiments are described in sufficient details for those skilled in the art to implement the present disclosure, but it should be understood that other implementations and aspects are possible, and changes in structures and structures and replacement of various elements are possible without departing from the scope and spirit of the technical idea of the present disclosure. Therefore, the following description is not to be construed as being limited thereto.

### (1) First Embodiment

### <Hardware Structure Example of Information Processing System>

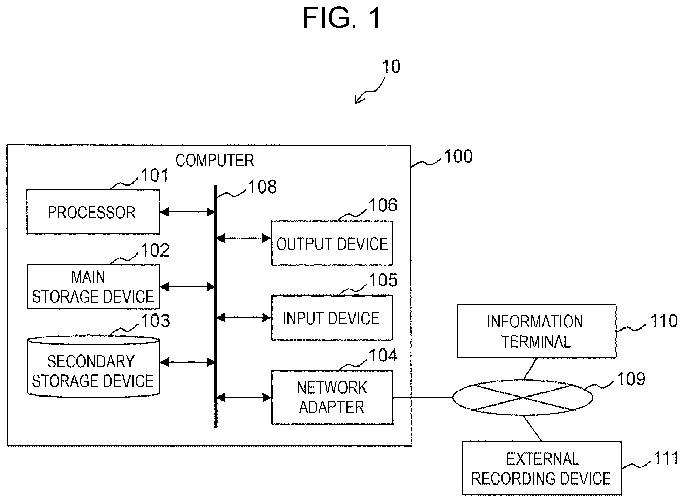

FIG. 1 is a diagram illustrating a schematic structure example of an information processing system (also referred to as a patient care plan determination system) 10 according to the present embodiment. A hardware structure example of the information processing system 10 is common to a first embodiment and a second embodiment to be described later.

The information processing system (patient care plan determination system) 10 includes, for example, a computer 100 provided on a cloud, an information terminal 110 installed in an examination room of a doctor, and an external recording device (medical information server) 111, which are connected via a network (intra-hospital network) 109.

The computer 100 includes a processor 101 such as a CPU, a main storage device 102, a secondary storage device 103, a network adapter 104, an input device 105, and an output device 106. The main storage device 102 stores, for example, a program for determining a test order to be described later. The secondary storage device 103 stores, for example, information (electronic medical record information, receipt information, guideline information, and the like) acquired from the external recording device 111. The processor 101 reads the program from the main storage device 102, loads the program in an internal memory (not illustrated), and implements each processing unit (various functions for determining a test order) to be described later.

The input device 105 includes a device that inputs information to the computer 100, such as a keyboard, a mouse, or a touch panel. The output device 106 includes a device that outputs information, such as a display or a printer. The network adapter 104 receives information from the information terminal 110 via the network 109 and transfers the information to the processor 101 or stores the information in the secondary storage device 103. The network adapter 104 transmits the determined test order information to the information terminal 110 via the network 109 in response to a command from the processor 101, for example.

### <Functional Configuration Example of Information Processing System>

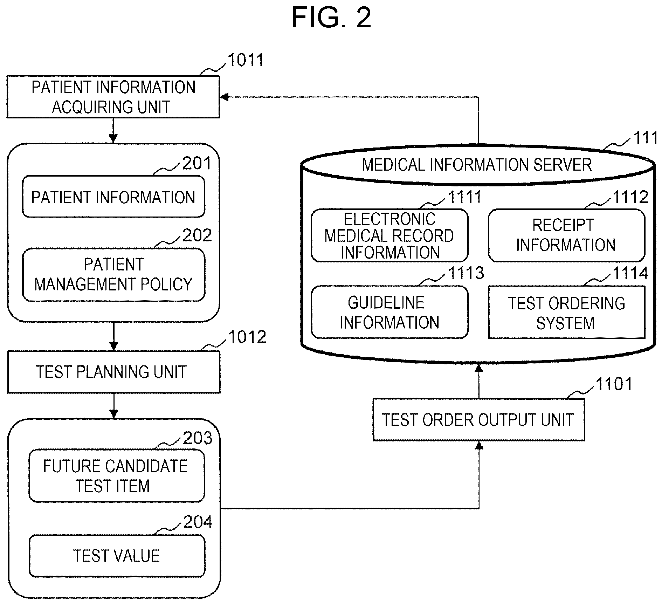

FIG. 2 is a diagram illustrating a functional configuration example (software configuration example) of the information processing system (patient care plan determination system) 10 according to the first embodiment.

The information processing system 10 includes, as functions, a test ordering system 1110 provided in the external recording device (medical information server) 111, a patient information acquiring unit 1011 and a test planning unit 1012 set in the processor 101 in the computer 100, and a test order output unit 1101 provided in the information terminal 110.

The information processing system 10 handles, as information, electronic medical record information 1111, receipt information 1112, and guideline information 1113 that are held in the external recording device (medical information server) 111, patient information 201 and a patient management policy 202, and a future candidate test item 203 and a test value 204. The receipt information 1112 includes medical service fee (point) information. The guideline information 1113 stores information on a test item medically determined according to symptoms (for example, a test X is performed for symptoms A and B). The patient management policy 202 is information indicating a patient classification such as an initial visit, a revisit, hospitalization, follow-up monitoring, and emergency.

The patient information acquiring unit 1011 accesses the electronic medical record information 1111 of the external recording device (medical information server) 111, and acquires, as the patient information 201, past test information and past diagnosis information corresponding to a target patient. The patient information acquiring unit 1011 acquires (receives) and outputs the patient management policy 202 input (selected) by a doctor from the information terminal 110. The patient information 201 includes, for example, patient attribute information (a gender, an age, and the like), a previously received test item and test result information, and patient disease information specified in the previous test. In this description, test result information at time point t (time step t) is represented as test result information t, and test result information at time point t+1 (time step t+1) is represented as test result information t+1. The disease information definitively diagnosed by a test t+1 performed at time point t+1 (time step t+1) is represented as patient disease information t+1. When the disease is undetermined by a test t at time point t, the disease is specified by performing a test t+1.

The test planning unit 1012 uses the patient information 201 and the patient management policy to calculate the test value 204 indicating an information amount (probability that a disease can be specified by each test item) according to a disease type in consideration of the patient management policy, and specifies a test item having a large information amount (high probability value) as the future candidate test item 203. The test planning unit 1012 transmits the specified future candidate test item 203 to the information terminal 110 as recommended test information.

The test order output unit 1101 receives the future candidate test item 203 and displays the future candidate test item 203 on a display screen (not illustrated) of the information terminal 110. When a doctor checks the future candidate test item 203 displayed on the display screen and selects a necessary test item from the future candidate test item 203, the test order output unit 1013 transmits the test order to the test ordering system 1110.

The test ordering system 1110 issues the test order received from the information terminal 110 to a test room.

### <Data Flow of Training Processing Executed by Test Training Unit 300 of Test Planning Unit 1012>

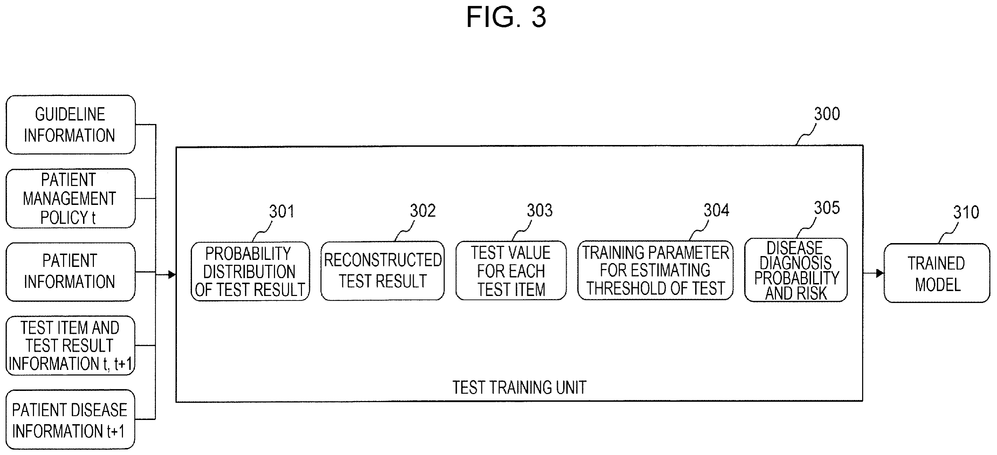

FIG. 3 is a diagram illustrating a data flow in training processing executed by a test training unit 300 of the test planning unit 1012. The test planning unit 1012 includes the test training unit 300 that generates a trained model and a test prediction unit 400 that infers a test item or the like using the trained model. In FIG. 3, the test training unit 300 generates various pieces of data and outputs a trained model 310 generated based on the data.

When generating a trained model, the test training unit 300 acquires, as input data, guideline information, a patient management policy t at time point t (time step t), patient attribute information, test item and test result information t at time point t and test result information t+1 at time point t+1 (time step t+1), and patient disease information t+1 at time point t+1. The guideline information and the patient attribute information are fixed information when generating the trained model, but other pieces of information change at each time point. That is, the patient management policy t at time point t (for example, when a patient is an initial visit patient last time, this time the patient is a second-time patient, so that a management policy changes), the test result thereof at time point t, the test result information t+1 at next time point t+1, and the patient disease information t+1 determined based on the test result information t+1 are all information that changes from time to time. The test training unit 300 executes training using information at a plurality of time points in this way, and constructs the trained model 310.

The test training unit 300 aggregates data of the test result for each test item using a probability distribution model, and estimates a probability distribution 301 (estimates the probability distribution 301 of the test result). As the number of pieces of data increases, the probability distribution 301 approaches a normal distribution.

The test training unit 300 generates a probability distribution function by training based on the estimated probability distribution, and generates a reconstructed test result 302 using the probability distribution function.

Next, the test training unit 300 compares the reconstructed test result 302 with an input test result to estimate a test value 303 for each test item.

Further, the test training unit 300 estimates a training parameter 304 for estimating a threshold of a test value, that is, a threshold of a test value for specifying a test item, and stores the training parameter threshold 304 in the main storage device 102 or the secondary storage device 103 for each test item.

The test training unit 300 estimates a diagnosis probability for the disease information t+1 at time point t+1 and a risk of the disease (disease diagnosis probability and risk 305).

The test training unit 300 determines a training parameter and outputs the training parameter as the trained model 310. The training parameter includes, for example, a parameter of a neural network for extracting a feature from patient information, a parameter for probability distribution estimation, a parameter for test result reconstruction, and a parameter for test value estimation.

### <Details of Training Parameter Determination Processing>

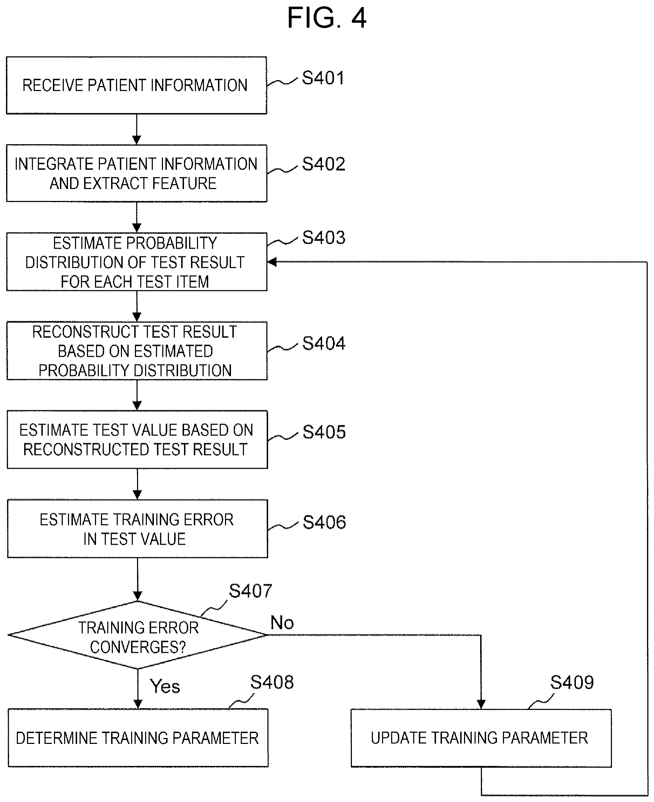

FIG. 4 is a flowchart illustrating details of training parameter determination processing according to the first embodiment. In the following description, an operation subject of each step is the test training unit 300, but since the test planning unit 1012 including the test training unit 300 is implemented by loading a program in the processor 101, the processor 101 may also be the operation subject.

### (i) S401

The test training unit 300 receives patient information necessary for generating the trained model 310. In order to generate the more accurate trained model 310, as many pieces of patient information as possible may be acquired. Here, the patient information includes various kinds of information on various patients, and as described above, each piece of information includes the guideline information, the patient management policy t at time point t (time step t), the patient information, the test item and test result information t at time point t and test result information t+1 at time point t+1 (time step t+1), and the patient disease information t+1 at time point t+1.

### (ii) S402

The test training unit 300 integrates the patient information acquired in S401 and extracts a feature of the patient information. Specifically, the test training unit 300 converts the received patient information into text and then into a numerical representation suitable for processing by a neural network (integrates the patient information), and applies the integrated patient information to a neural network (for example, deep learning) to calculate a feature of numerical information.

### (iii) S403

The test training unit 300 aggregates a plurality of test results for each test item, and estimates a probability distribution (function) for each test item using a probability distribution model.

### (iv) S404

The test training unit 300 reconstructs a test result based on the probability distribution estimated in S403. Since the test result as a correct value is already known, this processing verifies whether the correct value can be obtained from the estimated probability distribution.

### (v) S405

The test training unit 300 estimates a test value from the test result reconstructed in S404. When an original test result can be reconstructed from the estimated probability distribution, the estimated probability distribution is deemed to be correctly estimated. In S405, a test value, in other words, a value that is obtained when new patient information is input can be estimated.

### (vi) S406

The test training unit 300 estimates a training error in the test value estimated in S405. Here, the training error is an error between the original test result and the reconstructed test result.

### (vii) S407

The test training unit 300 determines whether a value of the training error estimated in S406 converges. Whether the value of the training error converges is determined based on whether the value of the training error is smaller than a preset threshold. When the value of the training error converges (Yes in S407), the processing proceeds to S408. When the value of the training error does not converge (No in S407), the processing proceeds to S409.

When the training error is small, a trained model capable of predicting a test value of a patient when new patient information is input can be constructed.

### (viii) S408

The test training unit 300 sets, into the trained model, a training parameter whose training error converges. Here, the training parameter includes the parameter of a neural network in S402, a parameter used in probability distribution estimation, a parameter used in test result reconstruction, and a parameter used in test value estimation.

### (ix) S409

The test training unit 300 updates the training parameter (for example, a parameter of a neural network) according to an optimization function for updating so as to reduce the training error.

### <Details of Processing of Determining Threshold>

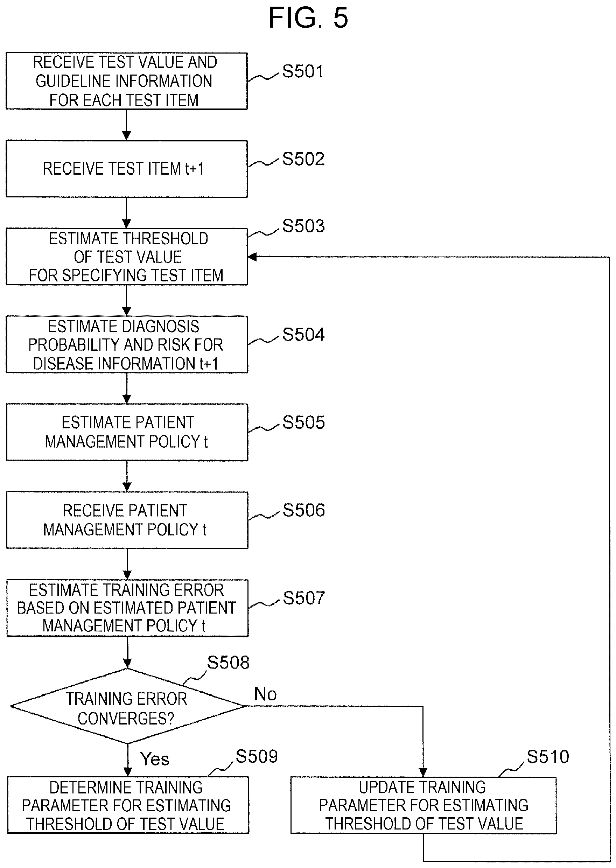

FIG. 5 is a flowchart illustrating details of processing of determining (training) a threshold of a test value when constructing a trained model.

### (i) S501

The test training unit 300 acquires a test value for each test item and the guideline information 1113.

### (ii) S502

The test training unit 300 receives the test item t+1 at time point t+1 (time step t+1).

### (iii) S503

The test training unit 300 estimates a threshold of the test value for specifying a test item. A training parameter for estimating the threshold of the test value can be determined by determining an initial value (fixed value) of the threshold and adjusting parameters to reduce the training error.

Since it is assumed that the threshold changes depending on a management policy (patient classification: an initial visit, a revisit, hospitalization, follow-up monitoring, emergency, and the like), the threshold of the test value is estimated in S503. The threshold is estimated for each test item, and a final threshold of the test value is determined (estimated) by training to match the management policy in subsequent processing.

### (iv) S504

The test training unit 300 estimates a diagnosis probability and the risk (a level of the risk of the diagnosed disease itself) for the disease information t+1 obtained from the test result t+1 corresponding to the test item t+1. Although the threshold estimated in S503 may not be accurate, a diagnosis probability and risk of a specific disease can be estimated using the test result reconstructed from the probability distribution estimated in the processing in FIG. 4 and the estimated threshold.

### (v) S505

The test training unit 300 estimates the management policy t at time point t (time step t) based on information on the diagnosis probability and risk estimated in S504. That is, in S505, it is estimated which classification (an initial visit, a revisit, hospitalization, follow-up monitoring, emergency, and the like) a patient belongs to.

### (vi) S506

The test training unit 300 acquires the actual patient management policy t.

### (vii) S507

The test training unit 300 compares the patient management policy t estimated in S505 with the actual patient management policy t acquired in S506 to estimate a training error.

### (viii) S508

The test training unit 300 determines whether a value of the training error estimated in S507 converges. Whether the value of the training error converges is determined based on whether the value of the training error is smaller than a preset threshold. When the value of the training error converges (Yes in S508), the processing proceeds to S509. When the value of the training error does not converge (No in S508), the processing proceeds to S510.

### (ix) S509

The test training unit 300 determines a parameter of the threshold of the test value estimated in S503 as a training parameter for estimating the threshold of the test value.

### (x) S510

The test training unit 300 updates the training parameter (parameter of a neural network) for estimating the threshold of the test value according to an optimization function. The processing of estimating the threshold of the test value for specifying the test item in S503 is executed again using the updated training parameter.

### <Data Flow of Prediction Processing Executed by Test Prediction Unit 600 of Test Planning Unit 1012>

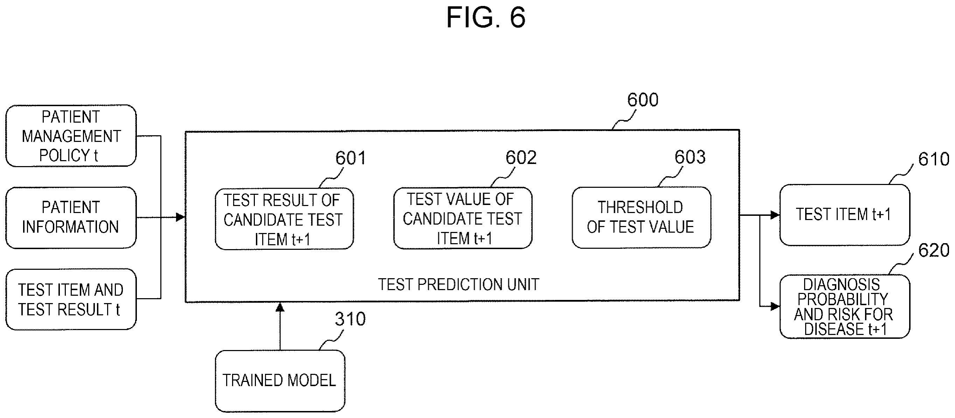

FIG. 6 is a diagram illustrating a data flow in prediction processing executed by a test prediction unit 600 of the test planning unit 1012. In FIG. 6, the test prediction unit 600 applies data at time point t (time step t) to the trained model, and outputs a test item t+1 at time point t+1 (time step t+1) and a diagnosis probability and risk for a disease t+1 at time point t+1.

When predicting the test item t+1 or the like, the test prediction unit 600 acquires, as input data, the patient management policy t at time point t, the patient attribute information, the test item and test result t at time point t. For example, based on the most recent patient management policy and the test result, a test item to be implemented at a next time point, a probability of being diagnosed with a specific disease, and a risk level of the disease are predicted.

The test prediction unit 600 applies the patient management policy t at time point t, the patient attribute information, and the test item and test result t at time point t to the trained model generated by the test training unit 300 to generate a candidate test item t+1 at time point t+1 and a test result 601 thereof, a test value 602 of each candidate test item t+1, and a threshold 603 in the test value. As described above, for example, the test item t may be a most recent test item, the test result t may be a most recent test result corresponding to the test item, and the candidate test item t+1 may be a candidate test item to be taken at the next time point.

The test prediction unit 600 further determines whether the test value 602 of the candidate test item t+1 (the test value of each candidate test item) is larger than the threshold 603 in the test value, determines the test item t+1 larger than the threshold, and estimates a probability (diagnosis probability) of being diagnosed with a disease at time point t+1 (disease at a next time point).

### <Details of Determination (Prediction) Processing of Test Item and the Like>

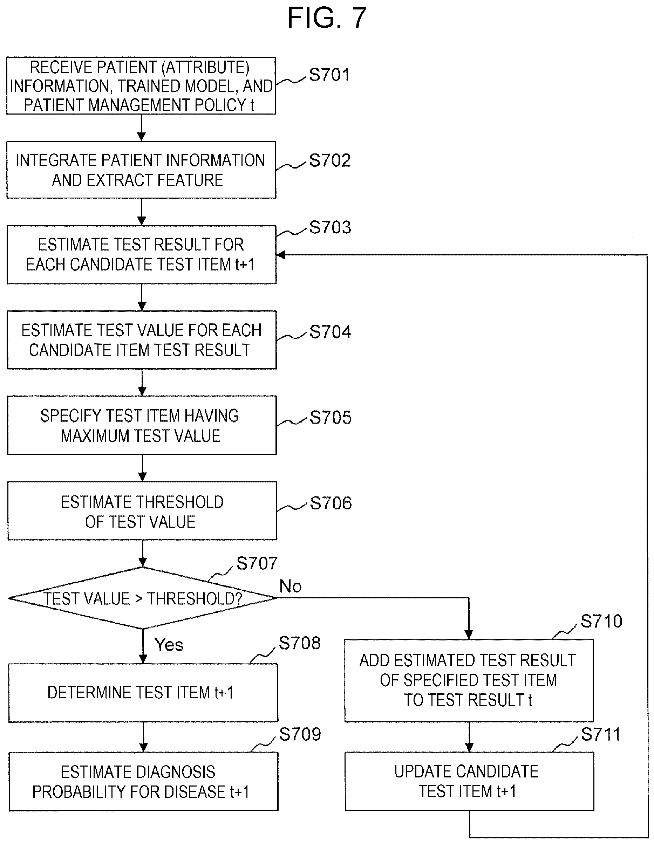

FIG. 7 is a flowchart illustrating details of determination of the test item t+1 at time point t+1 (time step t+1) and estimation of a probability of being diagnosed with the disease t+1 (diagnosis probability for the disease t+1) according to the first embodiment. In the following description, an operation subject of each step is the test prediction unit 600, but since the test planning unit 1012 including the test prediction unit 600 is implemented by loading a program in the processor 101, the processor 101 may also be the operation subject.

### (i) S701

The test prediction unit 600 receives (acquires), for example, patient information to be processed, the trained model 310, and the patient management policy t that are input by an operator.

### (ii) S702

The test prediction unit 600 integrates the patient information acquired in S701 and extracts a feature of the patient information. Specifically, the test prediction unit 600 converts the received patient information into text and then into a numerical representation suitable for processing by a neural network (integrates the patient information), and applies the integrated patient information to a neural network (for example, deep learning) to calculate a feature of numerical information.

### (iii) S703

The test prediction unit 600 estimates, for a plurality of test items, a test result by applying the feature extracted in S702 for each candidate test item t+1 to the trained model 310.

### (iv) S704

The test prediction unit 600 converts each test result estimated in S703 into a numerical representation to estimate the test value of each candidate test item t+1. The test value is a probability value indicating how much the diagnosis probability of the disease can be improved when the corresponding candidate test item is hypothetically selected. The test value is expressed as an information amount and is obtained by calculation by the trained model 310.

### (v) S705

The test prediction unit 600 specifies a test item having a maximum value among the test value estimated in S704.

### (vi) S706

The test prediction unit 600 estimates a threshold for determining the test value according to the management policy t and the information amount of the test value.

### (vii) S707

The test prediction unit 600 determines whether the test value specified in S705 is larger than the threshold estimated in S706. When the test value is larger than the threshold (Yes in S707), the processing proceeds to S708. When the test value is equal to or less than the threshold (No in S707), the processing proceeds to S710.

### (viii) S708

The test prediction unit 600 determines a test item having a maximum test value as a test item t+1_610.

### (ix) S709

The test prediction unit 600 estimates a probability and risk 620 of being diagnosed with the disease t+1 when the test item t+1 is implemented.

Specifically, the test prediction unit 600 reconstructs the test value from test value probability distribution information of the trained model 310 and estimates a disease diagnosis probability. Further, the test prediction unit 600 estimates a test value based on the estimated disease diagnosis probability. Then, the test prediction unit 600 specifies a test item having a maximum test value (disease diagnosis probability). The disease diagnosis probability corresponding to the specified test item is the probability of being diagnosed with the disease t+1. As described above, since the estimation of the test value is performed based on the disease probability estimation, the disease probability is also estimated in the process of the test value estimation. The test prediction unit 600 refers to the guideline information and specifies the risk (disease risk) 620 on a rule basis.

### (x) S710

The test prediction unit 600 adds the estimated test result of the test item specified in S705 to the test result t.

### (xi) S711

The test prediction unit 600 updates the candidate test item t+1. For example, when k candidate test items (m is a positive integer) are selected as the test item, a next group of the m candidate test items is set as a new test item to be processed. Then, the processing in S703 and subsequent processing are executed for the new candidate test item.

### <Example of Patient Information>

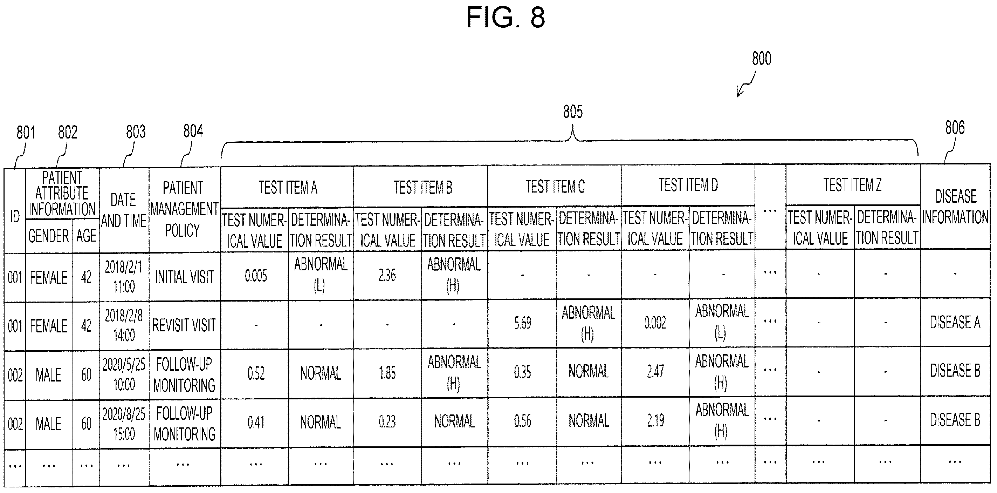

FIG. 8 is a diagram illustrating a configuration example of patient information 800 according to the present embodiment (common to the first embodiment and a second embodiment).

The patient information includes, as configuration items, identification information (ID) 801, patient attribute information 802, a date and time 803, a patient management policy 804, a result group 805 of a plurality of tests (test items A to Z), and disease information 806.

The identification information (ID) 801 is information for uniquely specifying and identifying a patient. The patient attribute information 802 is information indicating a sex and an age of the patient. The date and time 803 is information indicating a test date and time. The patient management policy 804 is information indicating patient classification such as an initial visit, a revisit, hospitalization, follow-up monitoring, and emergency, as described above. The result group 805 of a plurality of tests (test items A to Z) is information indicating a test numerical value and a determination result (abnormal (abnormal (H) due to an excessively high numerical value or abnormal (L) due to an excessively low numerical value), or normal) corresponding to each test item. The disease information 806 is information indicating a type of the disease diagnosed by a doctor based on the test result.

### <Test Cost Estimation Processing>

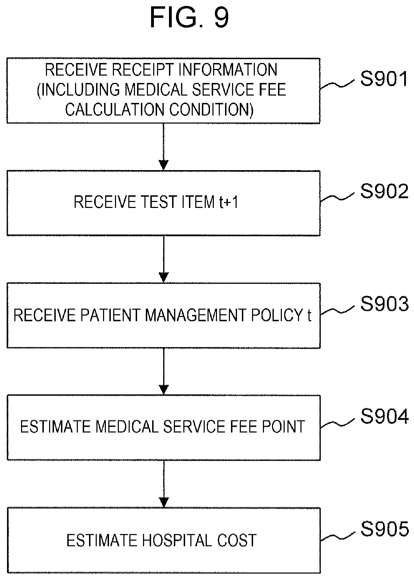

FIG. 9 is a flowchart illustrating test cost estimation processing executed by the test planning unit 1012. The test cost estimation processing is rule-based processing, and training is not performed.

### (i) S901

The test planning unit 1012 receives the receipt information 1112 including information on a medical service fee calculation condition.

### (ii) S902

The test planning unit 1012 receives information on the test item t+1_610 predicted by the test prediction unit 600.

### (iii) S903

The test planning unit 1012 receives the patient management policy t of a target patient.

### (iv) S904

The test planning unit 1012 estimates (calculates) a medical service fee point of the test item t+1_610 based on the medical service fee calculation condition acquired in S901.

### (v) S905

The test planning unit 1012 estimates (calculates) a hospital cost based on the medical service fee point estimated (calculated) in S904. Information on the estimated hospital cost can be transmitted to the information terminal 110 and displayed on the display screen of the information terminal 110.

### <Configuration Example of GUI Displayed on Display Screen of Information Terminal 110>

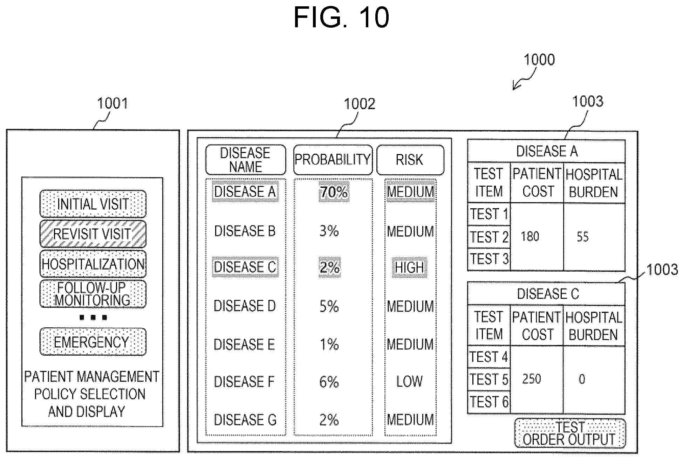

FIG. 10 is a diagram illustrating a configuration example of a test order GUI 1000 displayed on a display screen of the information terminal 110 according to the first embodiment. The patient management policy is selected and displayed (selected by the doctor) via the test order GUI 1000, and a disease determination result and a test item are output.

The test order GUI 1000 includes, as configuration items, for example, a management policy selection and display unit 1001, a disease probability display unit 1002, and a disease test item display unit 1003.

When recommended test information is provided to the computer 100 of the patient care plan determination system 10, the management policy selection and display unit 1001 displays a patient management policy of a target patient selectable by the doctor and a currently selected patient management policy.

The disease probability display unit 1002 provides information on a disease name, a disease probability, and a disease risk (risk degree of the disease itself) that are obtained by a test and disease prediction processing (prediction simulation). For example, a disease A obtained as a result of the prediction processing is applied with a probability of 70%, and it is indicated that a disease risk thereof is medium.

The disease test item display unit 1003 displays, for example, a test item for a disease having a disease probability higher than a predetermined value or a test item for a disease having a high risk, a patient cost indicating a cost paid by a patient for the test, and a hospital burden indicating a cost burden on the hospital. In the example in FIG. 10, test items of the disease A with a probability of 70% and a disease C with a high risk are displayed.

### (2) Second Embodiment

A second embodiment proposes the patient care plan determination system 10 that provides a medical service plan for a target patient in addition to the test plan according to the first embodiment. Since a hardware configuration of the patient care plan determination system 10 according to the second embodiment is as illustrated in FIG. 1, the description thereof is omitted.

### <Functional Configuration Example of Information Processing System>

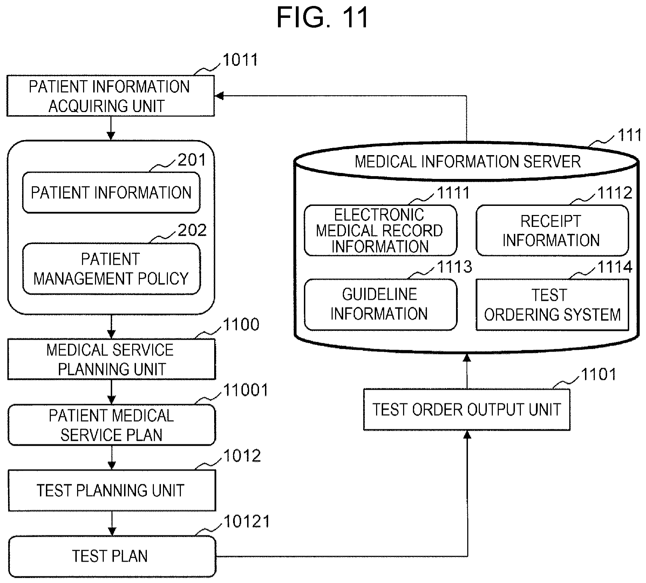

FIG. 11 is a diagram illustrating a functional configuration example (software configuration example) of the information processing system (patient care plan determination system) 10 according to the second embodiment.

The information processing system 10 includes, as functions, the test ordering system 1110 provided in the external recording device (medical information server) 111, the patient information acquiring unit 1011, a medical service planning unit 1100, and the test planning unit 1012 that are provided in the processor 101 in the computer 100, the information terminal 110, and the test order output unit 1101.

The information processing system 10 handles, as information, the electronic medical record information 1111, the receipt information 1112, and the guideline information 1113 that are stored in the external recording device (medical information server) 111, the patient information 201 and the patient management policy 202, and a test plan i_10121 (including, for example, the future candidate test item 203 and the test value 204 described above). The receipt information 1112 includes medical service fee (point) information. The guideline information 1113 stores information on a test item medically determined according to symptoms (for example, a test X is performed for symptoms A and B).

Similar to the first embodiment, the patient information acquiring unit 1011 accesses the electronic medical record information 1111 of the external recording device (medical information server) 111, and acquires, as the patient information 201, past test information and past diagnosis information corresponding to the target patient. The patient information acquiring unit 1011 acquires (receives) and outputs the patient management policy 202 input (selected) by a doctor from the information terminal 110. The patient information 201 includes information as illustrated in FIG. 8.

The medical service planning unit 1100 predicts (estimates) a future management policy, medical service method, and disease information using the patient information 201 and the patient management policy 202, and outputs them as a patient medical service plan 11001. For example, in a case of a hospitalized patient, the patient medical service plan 11001 includes information such as whether to continue the current treatment in the future and which method is used when the treatment method is changed.

As in the first embodiment, the test planning unit 1012 determines (predicts) a test plan 10121 using the patient information 201 and the patient management policy 202. As in the first embodiment, the test plan 10121 includes, as information, a future test item, a test result, and a test value prediction. The test plan 10121 includes the future candidate test item 203 and the test value 204 (see FIG. 2). The test planning unit 1012 calculates the test value 204 indicating an information amount (probability that a disease can be specified by each test item) according to a disease type in consideration of the patient management policy 202, and specifies a test item having a large information amount (high probability value) as the future candidate test item 203. The test planning unit 1012 transmits the specified future candidate test item 203 to the information terminal 110 as recommended test information.

The test order output unit 1013 receives the future candidate test item 203 and displays the future candidate test item 203 on a display screen (not illustrated). When the doctor checks the future candidate test item 203 displayed on the display screen and selects a necessary test item from the future candidate test item 203, the test order output unit 1013 transmits the test order to the test ordering system 1110.

The test ordering system 1110 issues the test order received from the information terminal 110 to a test room.

### <Detailed Internal Logical Configuration Example of Medical Service Planning Unit 1100 and Test Planning Unit 1012>

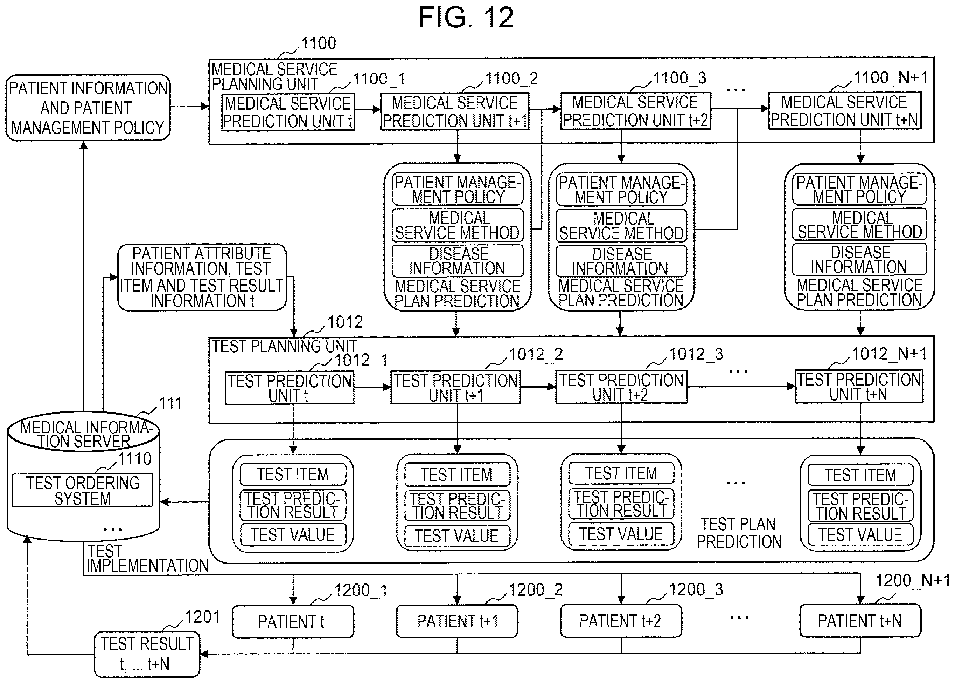

FIG. 12 is a diagram illustrating a detailed internal logical configuration example of the medical service planning unit 1100 and the test planning unit 1012 in the patient care plan determination system 10 of the second embodiment.

(i) The medical service planning unit 1100 includes N+1 medical service prediction units, that is, a medical service prediction unit t_1100_1 at time point t (time step t), a medical service prediction unit t+1_1100_2 at time point t+1 (time step t+1), a medical service prediction unit t+2_1100_3 at time point t+2 (time step t+2), and a medical service prediction unit t+1_1100_N+1 at time point t+N (time step t+N).

One medical service prediction unit t+k_1100_k+1 (k = 0, 1, ..., N) acquires (receives) a patient management policy and patient information (see FIG. 8) from time point t-k (time step t-k) to time point t (time step t) (acquires information before medical service prediction), uses the information to generate medical service plan prediction information including a patient management policy t+k, a medical service method t+k, and patient disease information t+k at time point t+k (time step t+k), and outputs the medical service plan prediction information to the test planning unit 1012.

(ii) The test planning unit 1012 includes N+1 test prediction units, that is, a test prediction unit t_1012_1 at time point t (time step t), a test prediction unit t+1_1012_2 at time point t+1 (time step t+1), a test prediction unit t+2_1012_3 at time point t+2 (time step t+2), and a test prediction unit t+1_1012_N+1 at time point t+N (time step t+N).

The test prediction unit t_1012_1 acquires patient information (FIG. 8), and test item and test result information t from the medical information server 111, and estimates a probability distribution of the test result for each test item → reconstructs the test result at next time step t+1 → estimates a test value, a disease diagnosis probability, and a risk thereof, as in the first embodiment. A test prediction unit t+k_1012_k+1 (k = 0, 1, ..., N) acquires patient information and test item and test result information (test prediction result estimated by a test prediction unit t+k-1) t+k at time point t+k (time step t+k), and estimates a probability distribution of the test result for each test item → reconstructs the test result at next time step t+k+1 → estimates a test value, a disease diagnosis probability and a risk thereof, and outputs the information.

(iii) When a test is actually executed, a patient test result 1200_1 at time point t (time step t), a patient test result 1200_2 at time point t+1 (time step t+1), a patient test result 1200_3 at time point t+2 (time step t+2), ..., and a patient test result 1200_N+1 at time point t+N (time step t+N) are output and aggregated, and stored in the medical information server 111 as test results t, ..., t+N_1201.

### <Data Flow of Training Processing Executed by Medical Service Training Unit 1301 of Medical Service Planning Unit 1100>

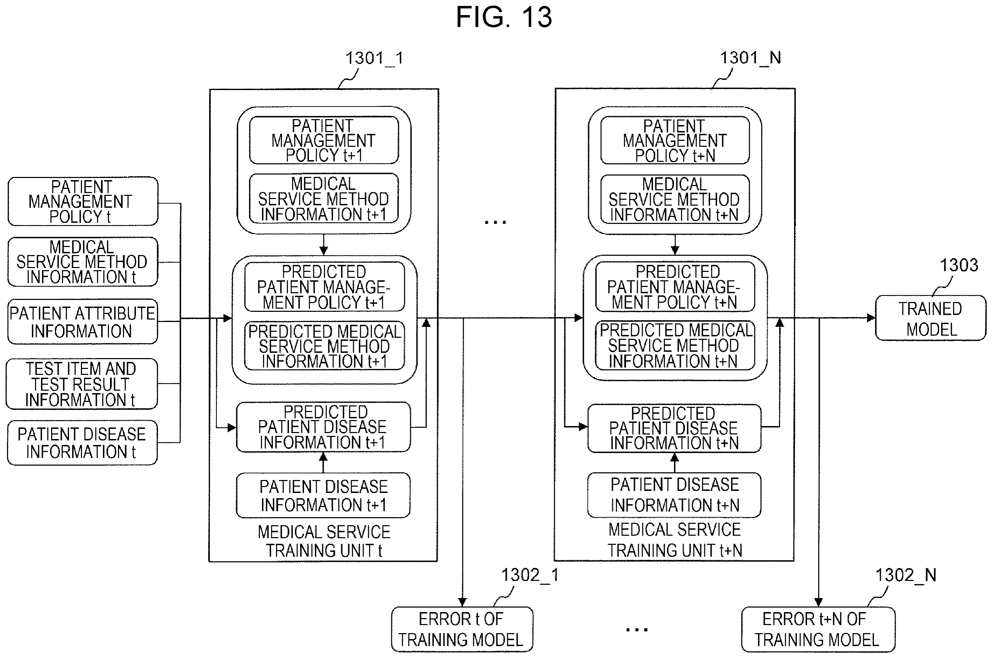

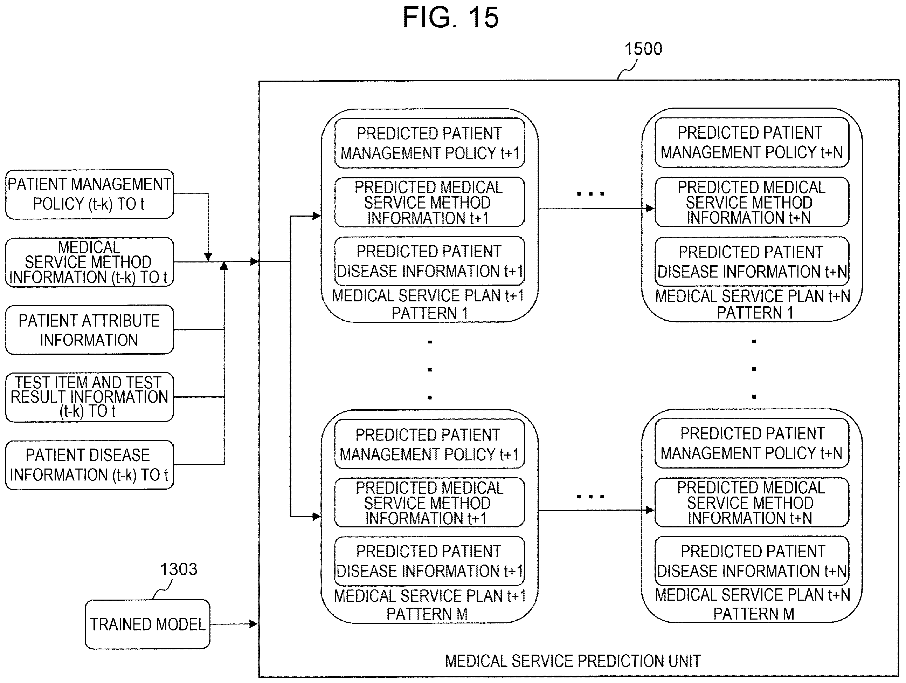

FIG. 13 is a diagram illustrating a data flow in training processing executed by a medical service training unit t1301_1 to a medical service training unit t+N1301_N of the medical service planning unit 1100. The medical service planning unit 1100 includes the medical service training unit t1301_1 to the medical service training unit t+N1301_N that generate a trained model, and a medical service prediction unit 1500 (see FIG. 15) that infers a medical service method or the like using the trained model. In FIG. 13, the medical service training unit t1301_1 to the medical service training unit t+N1301_N generate various pieces of data and output a trained model 1303 generated based on the data.

The medical service training unit t1301_1 acquires, as input data, a patient management policy at time point t (time step t), medical service method information t at time point t (time step t), patient attribute information, a test item, test result information t at time point t (time step t), and patient disease information t at time point t (time step t). The patient attribute information and the test item are fixed information when generating the trained model, but other pieces of information change at each time point. That is, the patient management policy t at time point t (time step t) (for example, when a patient is an initial visit patient last time, this time the patient is a second-time patient, so that a management policy changes), the test result thereof at time point t (time step t), the test result information t+1 at next time point t+1 (time step t+1), and the patient disease information t+1 determined based on the test result information t+1 are all information that changes from time to time. The medical service training units 1301_1 to 1301_N sequentially execute training using information at a plurality of time points in this way, and construct the trained model 1303.

A medical service training unit t1301 at time point t (time step t) and a medical service training unit t+k1301_k at time point t+k (k = 1, ..., N) (time step t+k) use the patient attribute information, patient management policies t and t+k at time point t and time point t+k (time step t and time step t+k), and medical service method information t+k to estimate a patient management policy t+k+1, a medical service method t+k+1, and patient disease information t+k+1 at time point t+k+1 (time step t+k+1). The medical service training unit t+k1301_k estimates an error t+k1302_k of the trained model by comparing an actual patient management policy t+k+1, an actual medical service method t+k+1, and an actual patient disease information t+k+1 with the predicted (estimated) patient management policy t+k+1, the predicted medical service method t+k+1, and the predicted patient disease information t+k+1. Further, the medical service training unit t+k1301_k converges an error t+k of the trained model by repeating the training processing, and determines a training parameter of the trained model 1303.

### <Trained Model Generation Processing Executed by Medical Service Training Unit>

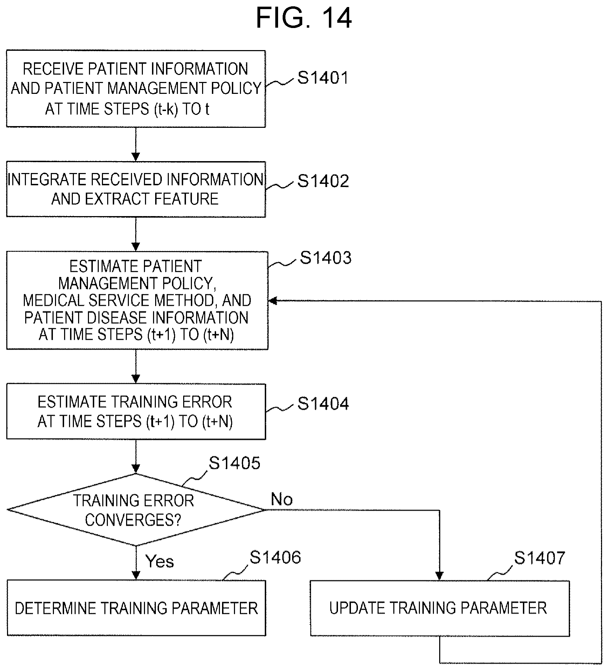

FIG. 14 is a flowchart illustrating training parameter determination processing (trained model generation processing) executed by the medical service training unit t1301_1 to the medical service training unit t+N_1301_N in the second embodiment. In the following description, an operation subject of each step is a medical service training unit, which is a general term for the medical service training unit t1301_1 or a medical service training unit t+k1301_k (k is an integer from 1 to N). The medical service planning unit 1100 including the medical service training unit 1301 is implemented by loading a program into the processor 101, and thus the processor 101 may also be the operation subject.

### (i) S1401

For example, the medical service training unit 1301 acquires, from the medical information server 111, information on a plurality of patients, that is, patient information and a patient management policy of the plurality of patients from time point t-k to time point t (time step t-k to time step t). Here, the patient information from time step t-k to time step t includes the patient attribute information, test item and test result information from time point t-k to time point t (time steps t-k to t), patient disease information, and medical service method information (see FIG. 8 for the configuration item of the patient information).

### (ii) S1402

The medical service training unit 1301 integrates the patient information and the patient management policy acquired in S1401, and extracts a feature of the information. Specifically, the medical service training unit 1301 converts the received patient information and patient management information into text and then into a numerical representation suitable for processing by a neural network (integrates the information), and applies the integrated information to a neural network (for example, deep learning) to calculate a feature of numerical information.

### (iii) S1403

The medical service training unit 1301 uses the feature extracted in S1402 to estimate a patient management policy, a medical service method, and patient disease information from time point t+1 to time point t+N (time step t+1 to time step t+N). For example, the estimation can be performed by machine learning using a neural network such as deep learning. For example, a patient management policy, a medical service method, and patient disease information at each time point in the future can be predicted by performing time-series learning on features such as patient information and a management policy at each time point in the past using a recurrent neural network (RNN) or the like. More specifically, a machine learning model used for the prediction calculation uses the patient information and the patient management policy at each time point in the past as time-series observation data, and predicts a change in patient disease information as a treatment effect by a specific medical service method.

### (iv) S1404

The medical service training unit 1301 compares the patient management policy, the medical service method, and the patient disease information (medical service plan) from time step t+1 to time step t+N that are acquired in S1403 with an actual patient management policy, an actual medical service method, and actual patient disease information from time step t+1 to time step t+N, and estimates a training error.

Here, the training error indicates an error in estimation results for a medical service plan pattern 1 to pattern M (M is an integer of 2 or more). The training error may be a weighted average of an actual error and a virtual error. The actual error represents, for example, a difference from patient information acquired based on a medical service plan (for example, a medical service plan 1) actually performed for the patient A. On the other hand, the virtual error represents a difference from virtual patient information acquired based on a medical service plan (for example, a medical service plan 2) that is not actually performed. The virtual patient information is acquired from another patient (for example, a patient B) who actually undergoes the medical service plan 2. The patient B is selected from a patient group having a feature of patient information similar to that of the patient A.

### (v) S1405

The medical service training unit 1301 compares the training error estimated in S1404 with a preset threshold, and determines whether the training error converges. When the training error converges (Yes in S1405), the processing proceeds to S1406. On the other hand, when the training error does not converge (No in S1405), the processing proceeds to S1407.

### (vi) S1406

The medical service training unit 1301 determines, as a parameter of the trained model, the parameter of the trained model used for the feature extraction in S1402, and the estimation of the patient management policy, the medical service method, and the patient disease information in S1403.

### (vii) S1407

The medical service training unit 1301 updates the training parameter according to an optimization function to reduce the training error. The updated training parameter is used in the repeated processing when re-estimating information such as a patient management policy.

### <Data Flow of Prediction Processing Executed by Medical Service Prediction Unit 1500 of Medical Service Planning Unit 1100>

FIG. 15 is a diagram illustrating a data flow in medical service prediction processing executed by the medical service prediction unit 1500 of the medical service planning unit 1100. The medical service prediction unit 1500 applies input data to the trained model 1303 to predict a tuberculosis medical service.

The medical service prediction unit 1500 acquires, as the input data, a patient management policy t-k at time point t-k (time step t-k) to a patient management policy t at time point t (time step t), medical service method information t-k at time point t-k (time step t-k) to medical service method information t at time point t (time step t), patient attribute information, test item and test result information t-k at time point t-k (time step t-k) to test result information t at time point t (time step t), and patient disease information t-k at time point t-k (time step t-k) to patient disease information t at time point t (time step t). In the second embodiment, a medical service plan t+1 at time point t+1 (time step t+1) to a medical service plan t+N at time point t+N (time step t+N) are predicted using information from time point t-k (time step t-k) to time point t (time step t) of a patient to be processed.

The medical service prediction unit 1500 calculates, for the medical service plan patterns 1 to M (M is an integer of 2 or more), a predicted patient management policy t+N, predicted medical service method information t+N, and predicted patient disease information t+N from a predicted patient management policy t+1, predicted medical service method information t+1, and predicted patient disease information t+1 by applying, to the trained model 1303, the patient management policy t-k at time point t-k (time step t-k) to the patient management policy t at time point t (time step t), the medical service method information t-k at time point t-k (time step t-k) to the medical service method information t at time point t (time step t), the patient attribute information, the test item and test result information t-k at time point t-k (time step t-k) to the test result information at time point t (time step t), and the patient disease information t-k at time point t-k (time step t-k) to the patient disease information t at time point t (time step t).

Here, the medical service plan patterns 1 to M are patterns obtained by extracting a possible medical service method for the target patient, and include not only the medical service method predicted based on the feature of the target patient but also a medical service plan pattern adopted for a patient having a feature similar to that of the target patient. This is to predict a treatment effect (disease information) of a treatment plan pattern that can be a treatment option, enabling a doctor to select an optimal treatment option.

### <Details of Determination (Prediction) Processing of Test Item and the Like>

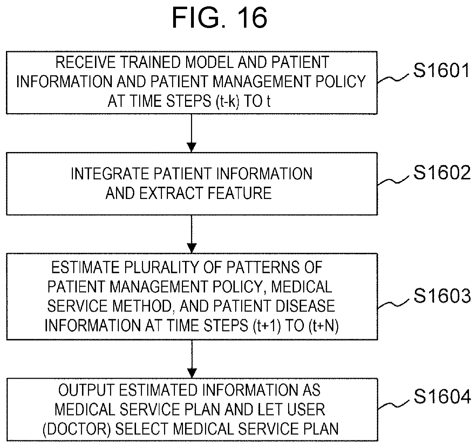

FIG. 16 is a flowchart illustrating processing of estimating a plurality of patterns of the patient management policy, the medical service method, and the patient disease information from time point t+1 (time step t+1) to time point t+N (time step t+N) according to the second embodiment. In the following description, an operation subject of each step is the medical service prediction unit 1500, but since the medical service planning unit 1100 including the medical service prediction unit 1500 is implemented by loading a program in the processor 101, the processor 101 may also be the operation subject.

### (i) S1601

For example, the medical service prediction unit 1500 acquires (receives), from the medical information server 111, patient information (patient information of a patient to be processed) from time step t-k to time step t and a patient management policy from time step t-k to time step t. The patient information from time step t-k to time step t is information including the patient attribute information, the medical service method information from time step t-k to time step t, the test result information from time step t-k to time step t, and the patient disease information from time step t-k to time step t. The medical service prediction unit 1500 sets up the trained model 1303 (prepares the trained model 1303 for use).

### (ii) S1602

The medical service prediction unit 1500 integrates the patient information acquired in S1601 and extracts a feature of the patient information. Specifically, the medical service prediction unit 1500 converts the received patient information into text and then into a numerical representation suitable for processing by a neural network (integration of the patient information), and applies the integrated patient information to a neural network (for example, deep learning) to calculate a feature of numerical information.

### (iii) S1603

The medical service prediction unit 1500 estimates, as a medical service plan, a patient management policy, patient disease information, and a medical service method from time step t+1 to time step t+N by applying the feature of the patient information acquired in S1602 to the trained model 1303.

The medical service plan is estimated in a plurality of patterns (pattern 1 to pattern M) for each time step from time step t+1 to time step t+N.

### (iv) S1604

The output device 106 receives, from the medical service prediction unit 1500, the information estimated in S 1603 (the patient management policy and the medical service method for each piece of patient disease information from time step t+1 to time step t+N), and transmits the information to the information terminal 110 as the medical service plan (pattern 1 to pattern M).

The information terminal 110 displays, for example, information on the received medical service plan on the display screen such that a user (doctor) can select a desired medical service plan.

### <Data Flow of Training Processing Executed by Test Training Unit 1700 of Test Planning Unit 1012>

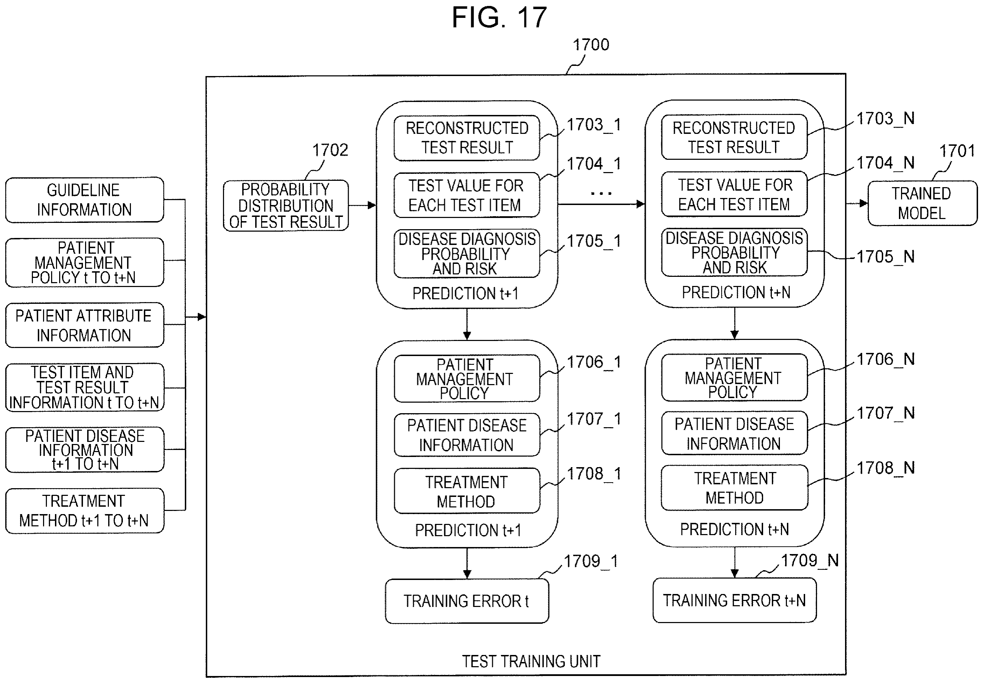

FIG. 17 is a diagram illustrating a data flow in training processing executed by a test training unit 1700 of the test planning unit 1012 of the second embodiment. The test planning unit 1012 includes the test training unit 1700 that generates a trained model and the test prediction unit 400 that infers a test item or the like using the trained model. In FIG. 17, the test training unit 1700 generates various pieces of data and outputs a trained model 1701 generated based on the data.

When generating a trained model, the test training unit 1700 acquires, as input data, guideline information, a patient management policy t at time point t (time step t) to a patient management policy t+N at time point t+N (time step t+N), patient attribute information, test item and test result information t at time point t (time step t) to test result information t+N at time point t+N (time step t+N), test result information t+1 at time point t+1 (time step t+1) to test result information at time point t+N (time step t+N), patient disease information t+1 at time point t+1 (time step t+1) to patient disease information t N at time point t+N (time step t+N), and a treatment method t+1 at time point t+1 (time step t+1) to a treatment method t+N at time point t+N (time step t+N).

The test training unit 1700 aggregates data of the test result for each test item using a probability distribution model, and estimates a probability distribution 1702 in the test result (estimates the probability distribution of the test result). As the number of pieces of data increases, the probability distribution approaches a normal distribution.

The test training unit 1700 generates a probability distribution function by training based on the estimated probability distribution 1702, and uses the probability distribution function to generate a reconstructed test result 1703_1 at time point t+1 (time step t+1) to a reconstructed test result 1703_N at time point t+N (time step t+N), a test value 1704_1 for each test item at time point t+1 (time step t+1) to a test value 1704_N for each test item at time point t+N (time step t+N), a disease diagnosis probability and risk 1705_1 at time point t+1 (time step t+1) to a disease diagnosis probability and risk 1705_N at time point t+N (time step t+N). From these pieces of information, the test training unit 1700 estimates a patient management policy 1706_1 at time point t+1 (time step t+1) to a patient management policy 1706_N at time point t+N (time step t+N), patient disease information 1707_1 at time point t+1 (time step t+1) to patient disease information 1707_N at time point t+N (time step t+N), and a treatment method 1708_1 at time point t+1 (time step t+1) to a treatment method 1708_N at time point t+N (time step t+N). Further, the test training unit 1700 compares the estimated patient management policy, patient disease information, and treatment method with an actual patient management policy, actual patient disease information, and an actual treatment method, and estimates a training error 1709_1 at time point t (time step t) to a training error 1709_N at time point t+N (time step t+N).

When the training error t to the training error t+N converges, the test training unit 1700 outputs the trained model 1701 having a parameter related to the converged training error.

### <Details of Training Parameter Determination Processing>

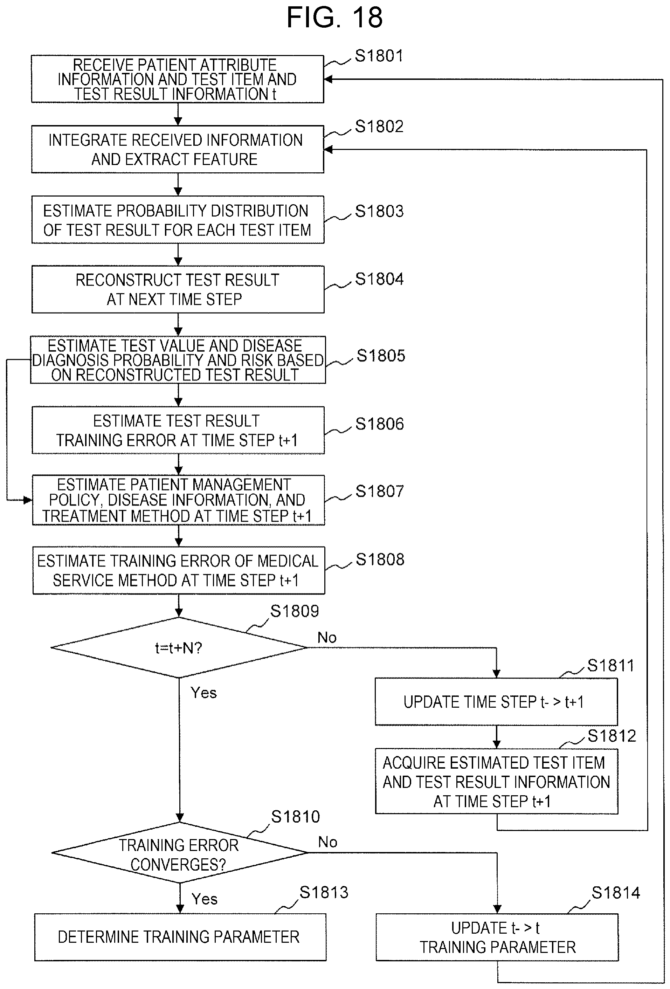

FIG. 18 is a flowchart illustrating details of training parameter determination processing according to the second embodiment. In the following description, an operation subject of each step is the test training unit 300, but since the test planning unit 1012 including the test training unit 1700 is implemented by loading a program in the processor 101, the processor 101 may also be the operation subject.

### (i) S1801

The test training unit 1700 receives patient attribute information necessary for generating the trained model 1701, test item information, and test result information t at time point t (time step t).

### (ii) S1802

The test training unit 1700 integrates the information acquired in S1801 and extracts a feature of the information. Specifically, the test training unit 1700 converts the received patient attribute information, test item information, and test result information t at time point t (time step t) into text and then into a numerical representation suitable for processing by a neural network (integrates the patient information), and applies the integrated information to a neural network (for example, deep learning) to calculate a feature of numerical information.

### (iii) S1803

The test training unit 1700 aggregates a plurality of test results for each test item, and estimates a probability distribution (function) for each test item using the probability distribution model.

### (iv) S1804

The test training unit 1700 reconstructs a test result at a next time step (t → t+1) based on the probability distribution estimated in S1703. Since the test result as a correct value is already known, this processing verifies whether the correct value can be obtained from the estimated probability distribution.

### (v) S1805

The test training unit 1700 estimates a test value, and a disease diagnosis probability and risk based on the test result reconstructed in S 1804. When an original test result can be reconstructed from the estimated probability distribution, the estimated probability distribution is deemed to be correctly estimated. In S1805, a test value, in other words, a value that is obtained when new patient information is input, and a disease diagnosis probability and risk can be estimated.

### (vi) S1806

The test training unit 1700 estimates a training error of the test result at a next time step (t → t+1) using the reconstructed test result estimated in S1804. Here, the training error is an error between an original test result and the reconstructed test result.

### (vii) S1807

The test training unit 1700 estimates a patient management policy, disease information, and a treatment method at the next time step (t → t+1) by applying the test value and the disease diagnosis probability and risk that are estimated in S1805 to, for example, a deep learning method (RNN) of time-series learning.

### (viii) S1808

The test training unit 1700 estimates a training error between an actual treatment method and the treatment method at the next time step (time step t+1) estimated in S1807.

### (ix) S1809

The test training unit 1700 determines whether the processing is completed for all time steps (t to t+N). When the processing is completed (Yes in S1809), the processing proceeds to S1810. When there is information on a time step that is not yet processed (No in S1809), the processing proceeds to S1811.

### (x) S1810

The test training unit 1700 determines whether a value of each of the training error estimated in S1806 and the training error estimated in S1808 converges. Whether the value of the training error converges is determined based on whether the value of the training error is smaller than, for example, a preset threshold. When the value of the training error converges (Yes in S1810), the processing proceeds to S1813. When the value of the training error does not converge (No in S1810), the processing proceeds to S1814.

### (xi) S1811

The test training unit 1700 updates a current time step (for example, t) to a next time step (for example, t+1).

### (xii) S1812

The test training unit 1700 acquires test item and test result information at current time step +1 (for example, t+1). Then, the processing proceeds to S1802, and integration of information and extraction of a feature are executed again.

### (xiii) S1813

The test training unit 1700 sets, into the trained model, a training parameter whose training error converges. Here, the training parameter includes a patient management policy, medical service method information, a neural network parameter inside RNN for predicting disease information, and the like.

### (xiv) S1814

The test training unit 1700 updates the training parameter (for example, a parameter of a neural network) according to an optimization function for updating to reduce the training error, and executes the processing from S1801 again.

### <Output Screen of Medical Service Plan and Test Plan>

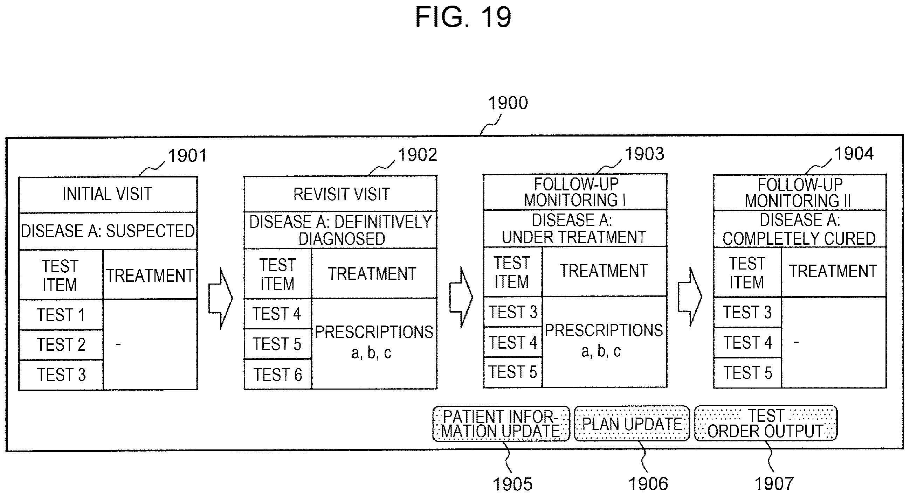

FIG. 19 is a diagram illustrating a configuration example of an output screen (user interface: UI) 1900 of a medical service plan and a test plan according to the second embodiment. The output screen 1900 can be displayed on a display screen of the information terminal 110, for example.

The output screen 1900 is a screen showing recommended contents of the medical service plan and the test plan, and specifically is a screen showing contents of the test item (estimated) and the treatment method (estimated) in a process from "suspected" to "cured" for a specific disease. The output screen 1900 includes, for example, an initial visit information display field 1901, a revisit information display field 1902, a follow-up monitoring information display field I_1903, a follow-up monitoring information display field II_1904, a patient information update button 1905, a plan update button 1906, and a test order output button 1907.

The patient information update button 1905 is a button to be pressed when, for example, a target patient changes and patient information of the target patient is to be input.

The plan update button 1906 is a button to be pressed when processing of creating (predicting) a medical service plan is executed again.

The test order output button 1907 is a button to be pressed when ordering a test to a test room (not illustrated) via the test ordering system 1110.

The output screen 1900 in FIG. 19 illustrates that when the disease A is suspected at an initial visit, a medical service plan and a test plan are predicted as follows: (i) at a revisit, a definitive diagnosis of the disease A is made, and tests 4, 5, and 6 are recommended, and prescriptions a, b, and c are also recommended as a treatment method; (ii) during a treatment, the tests 3, 4, and 5 are recommended, and the prescriptions a, b, and c are also recommended as the treatment method; and (iii) by continuing the tests and treatments shown in follow-up monitoring I, the patient A is completely cured.

### <Configuration Example of GUI Displayed on Display Screen of Information Terminal 110>

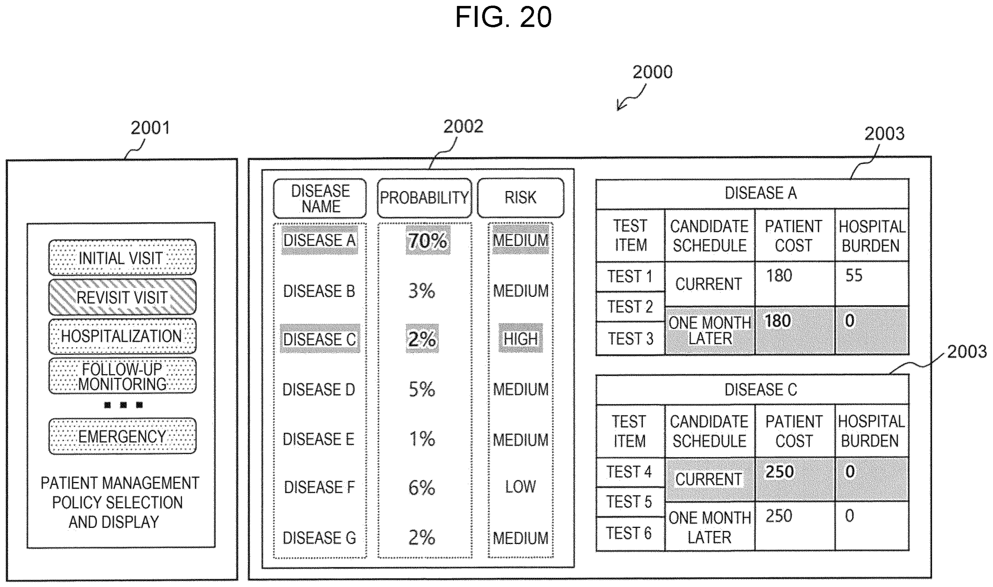

FIG. 20 is a diagram illustrating a configuration example of a test order GUI 2000 displayed on the display screen of the information terminal 110 according to the second embodiment. The patient management policy is selected and displayed (selected by the doctor) via the test order GUI 2000, and the disease determination result and the test item are output.

The test order GUI 2000 includes, as configuration items, for example, a management policy selection and display unit 1001, a disease probability display unit 2002, and a disease test item display unit 2003. A difference from the test order GUI 1000 according to the first embodiment is that the disease test item display unit 2003 of the test order GUI 2000 can allow comparison of a patient cost and a hospital burden at present and one month from now (future).

When recommended test information is provided to the computer 100 of the patient care plan determination system 10, the management policy selection and display unit 2001 displays a patient management policy of a target patient selectable by the doctor and a currently selected patient management policy.

The disease probability display unit 2002 provides information on a disease name, a disease probability, and a disease risk (risk degree of the disease itself) that are obtained by a test and disease prediction processing (prediction simulation). For example, a disease A obtained as a result of the prediction processing is applied with a probability of 70%, and it is indicated that a disease risk thereof is medium.

The disease test item display unit 2003 displays, for example, a test item for a disease having a disease probability higher than a predetermined value or a test item for a disease having a high risk, a patient cost indicating a cost paid by a patient for the test, and a hospital burden indicating a cost burden on the hospital. In the example in FIG. 10, test items of the disease A with a probability of 70% and a disease C with a high risk are displayed. For the disease A having a highest probability, it is illustrated that the patient cost is the same now and one month from now, whereas the hospital burden is 0 one month from now. On the other hand, for the disease C, which has a low probability but the highest risk, it is illustrated that there is no change in patient cost between now and one month from now, and the hospital burden is 0.

### (3) Summary

(i) The patient care plan determination system 10 according to the present embodiment is a system that uses a trained model to estimate a future candidate test item and a value (test value) thereof based on a patient management policy, patient information, and test item and test result information. As illustrated in FIGS. 1 and 2, the patient care plan determination system 10 may include the computer 100, the information terminal 110, and the medical information server (external recording device) 111. The computer 100 acquires predetermined information from the medical information server and the information terminal, and performs processing of determining a candidate test item related to a disease of a target patient. Specifically, the computer estimates the test value of the future candidate test item of the target patient by applying patient attribute information of the target patient, a test result of the target patient, and a patient management policy of the target patient to the trained model (test plan trained model) 310 for calculating a test value, which is a test value indicating information capable of identifying a disease, for each test item according to the patient management policy, and outputs the candidate test item of the target patient and the test value. An output destination can be, for example, the information terminal 110 operated by the user (doctor or the like). The candidate test item may be a test item having a maximum test value. In this way, a highly valid test can be recommended based on a result at each timing of testing, diagnosis, treatment, and follow-up monitoring within a holistic patient management workflow.
(ii) The test plan trained model 310 includes, as training parameters, a parameter for estimating a probability distribution of a test result for each test item, a parameter for reconstructing a test result based on the estimated probability distribution, and a parameter for estimating the test value. The outline of the training parameter generation processing is as follows. That is, the computer 100 extracts a feature by integrating patient information of a plurality of patients (training sample data), and estimates a probability distribution of the test result of the plurality of patients for each test item. The computer 100 generates a reconstructed test result by reconstructing the test result based on the estimated probability distribution, and estimates the test value based on the reconstructed test result. The computer 100 estimates a training error that is an error between an actual test value obtained from the test result and the estimated test value, and sets a training parameter for providing the training error as a training parameter of the trained model 310 when the training error converges. In this way, the trained model 310 for calculating information on a future candidate test item and a test value indicating a validity of the candidate test item by applying the information on a patient to be determined (the patient attribute information, the test result of the target patient, and the patient management policy of the target patient) can be constructed. The convergence of the training error can be determined using a threshold. When making the determination, it is necessary to use an appropriate threshold. Therefore, the computer 100 estimates a patient management policy using information on a diagnosis probability of a predetermined disease estimated based on the reconstructed test result and a risk of the disease, and estimates, based on a comparison result between the estimated patient management policy and an actual management policy, a threshold for determining whether the training error converges. Accordingly, the threshold used for the training error convergence determination can be set to an appropriate value.
(iii) The computer 100 estimates a medical service fee point based on the candidate test item, the patient management policy of the target patient, and a medical service fee calculation condition acquired from the medical information server, and estimates a hospital cost based on the medical service fee point. In this way, a future test item can be determined in consideration of cost-effectiveness.
(iv) In the second embodiment, the patient care plan determination system 10 that provides not only a test plan but also a medical service plan will be described. Specifically, the computer 100 acquires, from the medical information server 111, patient test and medical service history information including a patient management policy of the target patient in the past (at time step t-k) and the current time (at time step t), medical service method information of the target patient in the past and the current time, the patient attribute information of the target patient, test item and test result information of the target patient in the past and the current time, and patient disease information of the target patient in the past and the current time, and estimates a medical service plan including a patient management policy and a medical service method of the target patient in the future (at time step t+1 to time step t+N) by applying the patient test and medical service history information to the trained model (medical service plan trained model) 1303 for predicting a medical service plan of the target patient based on the patient test and medical service history information of the target patient. In this way, in addition to an appropriate test, the doctor can propose an appropriate treatment method at each time point in the future to the patient (even when an experience level of the doctor is poor). The computer 100 estimates a plurality of patterns of the medical service plan of the target patient at each future time point (time step t+1 to time step t+N) (see FIG. 15). Then, the information terminal 110 displays the information on the medical service plan of the target patient received from the computer 100 on the display screen in time series. For example, the information on the medical service plan is displayed on the display screen in the order of an initial visit, a revisit, and follow-up monitoring.
(v) The outline of the processing of generating a training parameter of the trained model 1303 is as follows. That is, the computer 100 acquires, from the medical information server 111, as patient test and medical service history information for trained model generation, a patient management policy of the plurality of patients (samples for trained model generation) before a predetermined time point (at time step t-k to time step t), medical service method information of the plurality of patients before the predetermined time point, the patient attribute information of the plurality of patients, test item and test result information of the plurality of patients before the predetermined time point, and patient disease information of the plurality of patients before the predetermined time point, extracts a feature of the information, performs machine learning based on the feature, and estimates a patient management policy, a medical service method, and patient disease information after the predetermined time point (at time step t+1 to time step t+N). The computer 100 estimates a training error for a medical service plan by comparing an actual patient management policy, medical service method, and patient disease information of the plurality of patients after the predetermined time point with the estimated patient management policy, medical service method, and patient disease information after the predetermined time point, and sets a training parameter for providing a training error for the medical service plan as a training parameter of the trained model 1303 when the training error converges. When the training error does not converge, the computer 100 updates the training parameter for providing the training error for the medical service plan using an optimization function, and executes the processing of estimating again using the updated training parameter. In this way, the trained model 1303 for appropriately proposing the future medical service plan by applying information on a patient for whom the medical service plan is to be created (the patient attribute information, the test result of the target patient, and the patient management policy of the target patient) can be constructed.
(vi) Functions of the present embodiment and each example can also be implemented by a software program code. In this case, a storage medium that records the program code is provided in a system or a device, and a computer (or CPU or MPU) of the system or the device reads the program code stored in the storage medium. In this case, the program code read from the storage medium implements the functions of the above-described embodiment, and the program code and the storage medium that stores the program code constitute the present disclosure. Examples of the storage medium for supplying such program codes include a flexible disk, a CD-ROM, a DVD-ROM, a hard disk, an optical disk, a magneto-optical disk, a CD-R, a magnetic tape, a nonvolatile memory card, and a ROM.

An operating system (OS) or the like operating on a computer may perform a part or all of actual processes based on an instruction of the program codes, and the functions of the embodiments described above may be implemented by the processes. Further, after the program codes read from the storage medium are written in a memory on the computer, a CPU or the like of the computer may perform a part or all of actual processes based on an instruction of the program codes, and the functions of the embodiments described above may be implemented by the processes.

The software program codes for implementing the functions of the embodiments and each example may be stored, by distributing via a network, in a storage unit such as a hard disk or a memory of the system or the device or a storage medium such as a CD-RW or a CD-R, and the computer (or CPU or MPU) of the system or the device may read and execute the program codes stored in the storage unit or the storage medium at the time of use.

The process and technique described here are not essentially related to any specific device and can be implemented by a combination of each component. In addition, various types of general-purpose devices can be added. A dedicated device may be constructed to execute the functions of the present embodiment and each example. In addition, various functions can be formed by appropriately combining a plurality of components disclosed in the present embodiment and each example. For example, some components may be deleted from all the components shown in the embodiment and each example, or components in different examples may be appropriately combined.

In the present disclosure, specific examples are described, but these are for description (understanding of the technology of the present disclosure) without limitation in all viewpoints. Those skilled in the art will readily recognize that there are numerous combinations of hardware, software, and firmware that are suitable for implementing the techniques of the present disclosure. For example, the above-described software can be implemented in a wide range of programs or script languages such as an assembler, C/C++, Perl, Shell, PHP, and Java (registered trademark).

Further, control lines and information lines considered to be necessary for description are shown in the embodiments described above, and not all control lines and information lines in a product are necessarily shown. All configurations may be connected to one another.

In addition, those skilled in the art can clarify other implementations of the present disclosure from consideration of the present embodiment and each example. The description and the specific examples are merely typical, and the scope and spirit of the technology of the present disclosure are indicated by the following claims.

### Reference Signs List

10 patient care plan determination system
100 computer
101 processor
102 main storage device
103 secondary storage device
104 network adapter
105 input device
106 output device
109 network
110 information terminal
111 external recording device, medical information server
1011 patient information acquiring unit
1012 test planning unit
1101 test order output unit
1100 medical service planning unit
1110 test ordering system

## Claims

1. A patient care plan determination system for determining at least a candidate test item related to a disease of a target patient, the system comprising:
a medical information server configured to store at least attribute information and a test result of a plurality of patients;
an information terminal configured to provide a patient management policy indicating a classification of the target patient; and
a computer configured to acquire predetermined information from the medical information server and the information terminal and determine the candidate test item related to the disease of the target patient, wherein
the computer executes
processing of acquiring, from the medical information server, attribute information of the target patient and a patient management policy of the target patient,
processing of estimating a test value of a future candidate test item of the target patient by applying the patient attribute information of the target patient, a test result of the target patient, and the patient management policy of the target patient to a test plan trained model for calculating the test value for each test item according to the patient management policy, the test value indicating information capable of specifying a disease, and
processing of outputting the candidate test item of the target patient and the test value.

2. The patient care plan determination system according to claim 1, wherein
the computer sets a test item having a maximum test value as the candidate test item of the target patient.

3. The patient care plan determination system according to claim 1, wherein
the test plan trained model includes, as training parameters, a parameter for estimating a probability distribution of a test result for each test item, a parameter for reconstructing a test result based on the estimated probability distribution, and a parameter for estimating the test value.

4. The patient care plan determination system according to claim 3, wherein
the computer executes
processing of acquiring, from the medical information server, patient information of the plurality of patients, the patient information including the patient attribute information, the test result, and the patient management policy,
processing of extracting a feature by integrating the patient information of the plurality of patients,
processing of estimating the probability distribution of the test result of the plurality of patients for each test item,
processing of generating a reconstructed test result by reconstructing the test result based on the estimated probability distribution,
processing of estimating the test value based on the reconstructed test result,
processing of estimating a training error that is an error between an actual test value obtained from the test result and the estimated test value, and
processing of setting a training parameter for providing the training error as a training parameter of the test plan trained model when the training error converges.

5. The patient care plan determination system according to claim 4, wherein
the computer estimates a patient management policy using information on a diagnosis probability of a predetermined disease estimated based on the reconstructed test result and a risk of the disease, and estimates, based on a comparison result between the estimated patient management policy and an actual management policy, a threshold for determining whether the training error converges.

6. The patient care plan determination system according to claim 1, wherein
the computer further executes
processing of acquiring, from the medical information server, receipt information including information on a medical service fee calculation condition,
processing of estimating a medical service fee point based on the candidate test item, the patient management policy of the target patient, and the medical service fee calculation condition, and
processing of estimating a hospital cost based on the medical service fee point.

7. The patient care plan determination system according to claim 1, wherein
the computer further executes
processing of acquiring, from the medical information server, patient test and medical service history information including a past and current patient management policy of the target patient, past and current medical service method information of the target patient, the patient attribute information of the target patient, past and current test item and test result information of the target patient, and past and current patient disease information of the target patient,
processing of estimating a medical service plan including a future patient management policy and medical service method of the target patient by applying the past and current patient test and medical service history information of the target patient to a medical service plan trained model for predicting a medical service plan of the target patient based on the patient test and medical service history information of the target patient, and
processing of outputting the medical service plan of the target patient.

8. The patient care plan determination system according to claim 7, wherein
the computer further
executes processing of extracting a feature of the patient test and medical service history information, and
applies the feature of the patient test and medical service history information to the medical service plan trained model.

9. The patient care plan determination system according to claim 7, wherein
the computer estimates a plurality of patterns of the medical service plan of the target patient at each future time point.

10. The patient care plan determination system according to claim 7, wherein
the information terminal receives information on the medical service plan of the target patient from the computer and displays a content of the medical service plan on a display screen in time series.

11. The patient care plan determination system according to claim 10, wherein
the information terminal displays the content of the medical service plan on the display screen in order of an initial visit, a revisit, and follow-up monitoring.

12. The patient care plan determination system according to claim 7, wherein
the computer generates the medical service plan trained model by executing
processing of acquiring, from the medical information server, as patient test and medical service history information for trained model generation, a patient management policy of the plurality of patients before a predetermined time point, medical service method information of the plurality of patients before the predetermined time point, the patient attribute information of the plurality of patients, test item and test result information of the plurality of patients before the predetermined time point, and patient disease information of the plurality of patients before the predetermined time point,
processing of integrating the patient test and medical service history information for trained model generation to extract a feature for trained model generation,
processing of performing machine learning based on the feature for trained model generation and estimating a patient management policy, a medical service method, and patient disease information after the predetermined time point,
processing of estimating a training error for a medical service plan by comparing an actual patient management policy, medical service method, and patient disease information of the plurality of patients after the predetermined time point with the estimated patient management policy, medical service method, and patient disease information after the predetermined time point, and
processing of setting a training parameter for providing a training error for the medical service plan as a training parameter of the medical service plan trained model when the training error for the medical service plan converges.

13. The patient care plan determination system according to claim 12, wherein
when the training error for the medical service plan does not converge, the computer updates the training parameter for providing the training error for the medical service plan using an optimization function, and executes the processing of estimating again using the updated training parameter.

14. A patient care plan determination method for determining at lease a candidate test item related to a disease of a target patient, the method comprising:
acquiring, by a computer, attribute information of the target patient and a patient management policy of the target patient from a medical information server, the computer being configured to acquire predetermined information from the medical information server and an information terminal and determine the candidate test item related to the disease of the target patient, the medical information server being configured to store at least attribute information and a test result of a plurality of patients, the information terminal being configured to provide a patient management policy indicating a classification of the target patient;
estimating, by the computer, a test value of a future candidate test item of the target patient by applying the patient attribute information of the target patient, a test result of the target patient, and the patient management policy of the target patient to a test plan trained model for calculating the test value for each test item according to the patient management policy, the test value indicating information capable of specifying a disease; and
outputting, by the computer, the candidate test item of the target patient and the test value.
